Europäisches Patentamt

**European Patent Office**

Office européen des brevets

(11) Publication number: **0 087 187**
**A1**

(12) **EUROPEAN PATENT APPLICATION**

(21) Application number: 83200210.9

(22) Date of filing: 10.02.83

(51) Int. Cl.³: **C 07 C 45/62**
**C 07 C 49/417**

(30) Priority: 15.02.82 NL 8200563

(43) Date of publication of application:
31.08.83 Bulletin 83/35

(84) Designated Contracting States:
AT BE CH DE FR GB IT LI NL

(71) Applicant: STAMICARBON B.V.
Postbus 10
NL-6160 MC Geleen(NL)

(72) Inventor: Thomissen, Petrus Jozef Hubertus
Planetenhof 41
NL-6215 TT Maastricht(NL)

(74) Representative: Pinckaers, August René et al,
OCTROOIBUREAU DSM Postbus 9
NL-6160 MA Geleen(NL)

(54) Process for the preparation of cyclohexyl-cyclohexanone.

(57) Disclosed is a process for the preparation of cyclohexyl cyclohexanone (CHA). Cyclohexanone is self-condensed to yield a mixture of cyclohexylidene cyclohexanone (CHDA), water, and unconverted cyclohexanone. The CHDA in the mixture is directly hydrogenated to CHA under an atmosphere of hydrogen and in the presence of a suitable catalyst, the presence of water and cyclohexanone not affecting the reaction. The CHA may be separated from the other components by, for example, fractional distillation following the hydrogenation.

EP 0 087 187 A1

# PROCESS FOR THE PREPARATION OF CYCLOHEXYL-CYCLOHEXANONE

The invention relates to a process for the preparation of cyclohexyl-cyclohexanone (hereinafter referred to as CHA) by hydrogenation in the liquid phase of cyclohexylidene-cyclohexanone (hereinafter referred to as CHDA), which is here also understood to mean the tautomer 2-(1-cyclohexenyl)-cyclohexanone, which hydrogenation is known from Japanese patent specification 47-16434.

The CHDA to be hydrogenated in this process can be obtained by self-condensation of cyclohexanone according to various known methods, for instance by passing cyclohexanone through an acid ion-exchanger at 90 °C and subjecting the resulting reaction mixture to distillation at reduced pressure (see German patent specification 857,960). Similar methods for the practical realization of this self-condensation are known from Japanese patent specification 70-41377, French patent specification 2,186,456 and British patent specification 1,493,231.

The reaction mixture obtained in this self-condensation of cyclohexanone contains non-converted cyclohexanone, which is separated from the desired product by fractionated distillation. In this fractionated distillation the water formed in the selfcondensation is also removed, so that the CHDA is separated off in virtually pure form.

It has now been found that the hydrogenation of CHDA to CHA need not be started from CHDA in virtually pure form, but that it may just as well be started from the CHDA-containing mixture that is obtained in the self-condensation of cyclohexanone, and in which water and cyclohexanone are still present, and that it is therefore not necessary first upgrading this mixture. In said hydrogenation water is not found to interfere and the cyclohexanone is not hydrogenated.

The process according to the invention for the preparation of CHA by hydrogenation in the liquid phase of CHDA is characterized in that it starts from a CHDA-containing mixture obtained by self-condensation of cyclohexanone, and also containing water and cyclohexanone, and that CHA and cyclohexanone are recovered from the hydrogenation mixture obtained.

The hydrogenation according to the invention can be effected at various temperatures, for instance temperatures of 10-350 °C, while applying hydrogenation catalysts known per se. By preference a temperature of 50-165 °C is applied, because this permits of working at atmospheric pressure in the liquid phase. Very suitable as hydrogenation catalyst are the noble metals of the 8th group of the periodic system of elements according to Mendeleev, particularly platinum and palladium, or a compound of these metals, for instance an oxide. These catalysts may be applied on a carrier, such as carbon, $SiO_2$, $MgO$ and $Al_2O_3$.

By adding an alcohol, for instance in an amount of 2-100 g alcohol per 100 g starting mixture, to the reaction mixture, the hydrogenation rate can be increased substantially. Suitable alcohols for this are, for instance, methanol, ethanol, propanol, isopropanol, butanol, hexanol and cyclohexanol. The alcohol to be used need not be water-free. Even with aqueous alcohol, for instance ethanol containing 50 % water, the hydrogenation rate can be increased substantially. The amount of alcohol chosen preferably is such that 10-50 g alcohol is present per 100 g of the mixture to be hydrogenated. Below the value of 10 g alcohol, the increase in the hydrogenation rate is slight, while above the value of 50 g the hydrogenation rate hardly increases further.

The partial pressure chosen for the hydrogenation may vary, for instance from 0.5 to 50 bar. By preference a partial hydrogen pressure of 0.5-1 bar is applied, as this obviates the need to work in pressure equipment.

The hydrogenation mixture obtained according to the invention may be upgraded in various ways, for instance by fractionated distillation. Thus, the desired product is obtained alongside with the alcohol that may have been applied, any non-converted CHDA and cyclohexanone. The alcohol, the CHDA and the cyclohexanone may, if desired, be applied again. The CHA obtained may be applied, for instance, in the fragrances industry.

The process according to the invention will be elucidated in the following examples.

Example I

Cyclohexanone is passed through a column (diameter 20 mm, height 180 mm) filled with 35 g strongly acid ion-exchanger (Lewatit SPC 118 W), the space velocity being 8 ml cyclohexanone per ml ion-exchanger (bulk volume) per hour. By means of a heating jacket of the column, the temperature in the column is kept at 90 °C. A reaction product is obtained which contains 88.6 wt. % cyclohexanone, 9.2 wt. % CHDA, 0.2 wt. % CHA and 2 wt. % water.

Using a 1-litre flask provided with a reflux cooler and an inlet tube for hydrogen, 600 g of said reaction product is, with stirring, hydrogenated with hydrogen (purity 99 %) in the presence of 245 g n-hexanol at a temperature of 75 °C, use being made of a palladium-on-Al$_2$O$_3$ catalyst (10 wt. % Pd, commercially available under the name Engelhard A 720085). The amount of palladium applied is 0.25 g per 100 g CHDA. The hydrogen is introduced at a rate of 0.7 standard litre per minute. After 1 hour, 83 % of the CHDA is found to be converted into CHA.

Example II

In the same way as in Example I, 600 g of said reaction product, in the presence of 165 g n-hexanol with a platinum-on-carbon catalyst (5 wt. % Pt, commercially available under the name Engelhard A 723315), is hydrogenated under otherwise unchanged conditions. After 1 hour, 91 % of the CHDA is found to be converted into CHA.

Example III

In the same way as in Example I, with the aid of the ion-exchanger at 110 °C and the same space velocity, cyclohexanone is converted into a reaction product containing 61.4 wt. % cyclohexanone, 31.8 wt. % CHDA, 0.8 wt. % CHA and 6 wt. % water. This reaction product is hydrogenated in the way set forth in Example I at a temperature of 70 °C with a palladium-on-carbon catalyst (5 wt. % Pd, commercially available under the name Engelhard A 722441).

600 g reaction product and 120 g ethanol (96 wt. %) are introduced into the flask. The amount of palladium applied is 0.25 g per 100 g CHDA. After 40 minutes the CHDA is found to have been converted virtually completely into CHA. The hydrogenation mixture is then separated off from the catalyst, and the separated catalyst is again applied for the hydrogenation of a subsequent batch of 600 g reaction product in the same way. After this, 50 more batches are hydrogenated in this way. The total amount of hydrogenation mixture (36.2 kg) is subjected to fractionated distillation at atmospheric pressure. This yields 6.9 kg of an ethanol-water mixture (72.5 wt. % ethanol), 19.1 kg cyclohexanone and 10.2 kg CHA (purity approximately 96 %) which is converted into 8.6 kg virtually pure CHA by distillation at reduced pressure (50 mbar).

CLAIMS

1. Process for the preparation of cyclohexyl-cyclohexanone by hydrogenation in the liquid phase of cyclohexylidene-cyclohexanone, characterized in that a cyclohexylidene-cyclohexanone-containing mixture, obtained by self-condensation of cyclohexanone and also containing water and cyclohexanone, is started from and cyclohexyl-cyclohexanone and cyclohexanone are recovered from the hydrogenation mixture obtained.

2. Process according to claim 1, characterized in that the hydrogenation is effected at a temperature of 50-165 °C.

3. Process according to either of claims 1 and 2, characterized in that the hydrogenation is effected with the aid of a hydrogenation catalyst containing a noble metal of the 8th group of the periodic system of the elements according to Mendeleev, or a compound of such a metal.

4. Process according to claim 3, characterized in that a hydrogenation catalyst is applied that contains platinum and/or palladium in the form of metal and/or in the form of a compound.

5. Process according to any one of the claims 1-4, characterized in that the hydrogenation is effected in the presence of an alcohol.

6. Process according to claim 5, characterized in that 10-50 g alcohol is applied per 100 g of the mixture to be hydrogenated.

7. Process according to any one of the claims 1-6, characterized in that the hydrogenation is effected while applying a partial hydrogen pressure of 0.5-1 bar.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | DE-C- 922 167 (BASF) <br> * Claim * | 1 | C 07 C 45/62 <br> C 07 C 49/417 |
| A | DE-A-2 705 857 (UNION CARBIDE) <br> * Page 35, example 12 * | 1 | |
| A | BERICHTE DER DEUTSCHEN CHEMISCHEN GESELLSCHAFT, vol. 75, 1942, pages 384-394, Berlin, DE. <br> J. REESE: "über 2-Cyclohexyliden-Cyclohexanon, ein Isomeres des 2-delta1-Cyclohexenyl-cyclohexanons" * Page 385, compound IV; pages 390-391 * | 1 | |
| A | CHEMICAL ABSTRACTS, vol. 53, 1959, column 2111g, Columbus, Ohio, USA <br> W. HÜCKEL et al.: "Stereospecificity of reactions" & IBID. 46-81, 1958 | 1 | TECHNICAL FIELDS SEARCHED (Int. Cl. ³) <br><br> C 07 C 45/00 <br> C 07 C 49/00 |
| A | EP-A-0 016 650 (NIPPON ZEON) <br> * Page 3 * | 1 | |

The present search report has been drawn up for all claims

| Place of search <br> THE HAGUE | Date of completion of the search <br> 09-05-1983 | Examiner <br> BONNEVALLE E.I.H. |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82